# EUROPEAN PATENT SPECIFICATION

(11) **EP 2 380 533 B1**
(45) Date of publication and mention of the grant of the patent: **25.11.2015**
(21) Application number: 09834165.4
(22) Date of filing: 22.12.2009
(51) Int. Cl.: A61F 5/56

(54) **INTRAORAL SPLINT**
INTRAORALE SCHIENE
ORTHÈSE BUCCALE

(30) Priority: 23.12.2008 ES 200901080 U
(43) Date of publication of application: 26.10.2011
(73) Proprietor: Navarro Segura, Miguel, 03680 ASPE (Alicante) (ES); Soria Alcaraz, Antonio, 03680 ASPE (Alicante) (ES); Mangada Martinez, Federico, 03680 ASPE (Alicante) (ES)
(72) Inventor: Navarro Segura, Miguel, 03680 ASPE (Alicante) (ES); Soria Alcaraz, Antonio, 03680 ASPE (Alicante) (ES); Mangada Martinez, Federico, 03680 ASPE (Alicante) (ES)
(74) Representative: Carpintero Lopez, Francisco
(86) International application number: PCT/ES2009/070617
(87) International publication number: WO 2010/072878

(56) References cited:
- WO-A1-2006/063403
- ES-U- 1 067 430
- US-A- 5 915 385
- US-A1- 3 132 647
- US-A1- 2008 041 396

## Description

### Object of the Invention

The object of the present invention patent is to provide a new intraoral splint with a rear arch to prevent snoring and apneas which prevents the possible shift of the tongue towards the rear part of the oral cavity of said user by being placed in the user's maxilla, thereby preventing the possible blockage of the airway due to the superposition of the rear part of the tongue or soft palate on the posterior pharyngeal wall.

The invention is particularly applicable in the otolaryngology sector where there is a need to correct those problems.

### Background of the invention

Until now, various devices have been disclosed in the current state of the art to solve snoring and apnea problems having specific technical features for each case.

Document EP-A-0,312,368 comprises an intraoral device to prevent snoring. This device consists of a U-shaped member formed to the shape of the user's upper dental arch and includes an inclined lower ramp for engaging with the teeth in the mandible. The normal movements of the mouth, such as for example closing the mandible and maxilla, will cause part of the set of teeth in the mandible to engage the lower part of the ramp, which cams the mandible forward to increase the gap between the base of the tongue and the posterior pharyngeal wall.

Said device is clearly different from the one described in the present invention patent.

Invention patent ES 2217284 T3 discloses a device to prevent the stertorous breathing or snoring which is adapted to be fixed in the upper part of a person's mouth, comprising a non-rigid flexible transverse bridge for being fixed in the upper part of the mouth and a member (for gripping the tongue) extending downwards from said non-rigid flexible transverse bridge.

The main difference with respect to said invention patent is that rather than gripping the tongue by default and in a fixed manner, the present invention is provided with a rear arch for simply being able to limit the movement of the tongue in the event of a possible shift thereof, and it can therefore be maintained in a position where it does not block the airway due to the superposition of the rear part of the tongue or soft palate on the posterior pharyngeal wall.

It should also be mentioned that the present invention is based on a rear arch, not a transverse bridge.

Patent ES 2118365 T3 discloses a dental apparatus for treating snoring and obstructive sleep apnea episodes. This apparatus made to be worn by a patient while sleeping has an upper member formed to the shape of the set of teeth in the patient's maxilla and connecting means for releasably coupling said upper and lower members to one another wherein said connecting means adjustably keep said lower member in an anterior position, projecting in relation to said upper member, and wherein said connecting member allows the lateral movement of said lower member in relation to said upper member in said projected position.

This dental apparatus differs from the present invention patent in that it is essentially based on positioning the upper member formed to the shape of the set of teeth in the maxilla of the patient with respect to the lower member formed to the shape of the set of teeth of the mandible of the patient by means of a connector which positions said members and therefore allows adjusting said positions with respect to one another.

Utility Model U200800413 discloses an intraoral device to prevent snoring and apneas. Said device is made up of a body fitted in the user's maxilla and exerting uniform pressure thereon, said body incorporating in its rear part a flap, which may vary in shape and size according to the morphology of the user's mouth.

The differences with respect to the present invention are significant because it relates to a palate provided with a flap which is driven into a certain point of the tongue, causing long-term damage therein as well as complicating proper salivation.

Likewise, the patent Application US05915385 A describes a dental device intended to eliminate snoring and relieve stress, the device comprising an upper member configured as a U-shaped mouthpiece for engaging at least some of the teeth of the upper dentition of a patient or user.

Although the dental apparatuses of the state of the art have proven to be effective to keep the mandible in a projected position to improve the clearance of the airway, they sometimes result in unwanted side effects. One of the most common side effects is damage to the temporomandibular joint and the maxillary muscles and related ligaments, particularly in individuals who tend to grind their teeth while sleeping. Damage to the temporomandibular joint has been associated with a wide range of physical diseases, including migraine-type headaches. Therefore, many individuals who suffer snoring and sleep apnea disorders are unable to tolerate the existing anti-snoring dental apparatuses for prolonged time periods.

This new intraoral splint with a rear arch to prevent snoring and apneas seeks to prevent the possible shift of the tongue towards the rear part of the oral cavity and thus eliminate the possible blockage of the airway due to the superposition of the rear part of the tongue or soft palate on the posterior pharyngeal wall.

At no time does the state of the art disclose an intraoral splint with a rear arch to prevent snoring and apneas such as the one described in the present invention patent.

### Description of the Invention

This new intraoral splint with a rear arch to prevent snoring and apneas, object of the present invention patent, made up of a splint of the type coupled to the user's teeth and incorporating an arch in its rear part, thereby preventing the possible blockage of the airway due to the superposition of the rear part of the tongue or soft palate on the posterior pharyngeal wall, is proposed to palliate or, where appropriate, eliminate all the drawbacks discussed above.

The most significant advantages provided with this new intraoral splint are:
- It can be customized for each person, varying both the shape of the splint and the size and angle of inclination of said arch.
- It can be custom-made from a single oval-shaped part that is neither supported on nor secured to the palate, allowing the opening of the airways and therefore without detriment to the creation of saliva accumulators, thereby suppressing one that may present said discomfort.
- Since the arch is as wide as the tongue, it allows homogenous contact thereof along the entire base.
- It prevents local friction and all that this entails; anxiety, excessive salivation, drooling while sleeping, small ulcers, etc.
- It leaves the tongue completely free in its rest state.

Adhesive is necessary only in the cases of people without teeth.

Said arch placed in the rear part can be divided into two halves, performing the same functionality because it prevents the tongue from shifting towards the rear part of the oral cavity. Since it is divided into two halves, said arch can facilitate the process of manufacturing the splint. The arch can likewise be a single continuous part and the splint can be split in order to be able to be adapted to different user mouth sizes.

In a preferred solution, said arch will be placed in the specific area between the canines or second molars, close to the tubercles, which is the boundary with the soft palate, and said boundary will depend on the morphology of the mouth.

The angle adopted by the rear arch ranges between 30 degrees and 120 degrees with respect to the axis of the splint itself, this range for the aforementioned angle of the arch being suitable for complying with the function of shifting the tongue towards the rear part of the oral cavity.

The size of the arch will depend on the person's palate and on the size of his teeth, size being understood as the separation between the branches of the aforementioned arch, which can range between 10 and 60 mm, such that it has no ridges or edges damaging the tissues. The diameter of the arch ranges between 1 mm and 20 mm, the section of the arch being able to be circular shaped, oval shaped or having any other suitable shape, ridges or sharp edges that may damage the tissues of the oral cavity being prevented with those sections. Said section can also be planar or flattened in order to be better adapted to the palate.

As described, the functionality of the arch consists of limiting the possible rearwards shift of the tongue, preventing the blockage of the airway due to the superposition of the rear part of the tongue or soft palate on the posterior pharyngeal wall.

### Description of the Drawings

To complement the description that is being made and for the purpose of aiding to better understand the features of the invention, a series of drawings is attached to the present specification as an integral part thereof in which the following has been depicted with an illustrative and non-limiting character:
Figure 1 shows a side view of the intraoral splint with a rear arch to prevent snoring and apneas.
Figure 2 shows a front view of the intraoral splint with a rear arch to prevent snoring and apneas.
Figure 3 shows a top view of the intraoral splint with a rear arch to prevent snoring and apneas.
Figure 4 shows a perspective view of the intraoral splint with a rear arch to prevent snoring and apneas.
Figure 5 shows a side sectional view of an application of the intraoral splint with a rear arch to prevent snoring and apneas, with the tongue in its normal rest state.
Figure 6 shows a side sectional view of an application of the intraoral splint with a rear arch to prevent snoring and apneas, with the tongue shifted and secured by means of said splint.
Figure 7 shows a perspective view of a splint and its rear arch made in a simplified form.
Figure 8 shows a side view of the splint of the preceding figure to show the angle formed between both.

### Preferred Embodiment of the Invention

This intraoral splint with a rear arch to prevent snoring and apneas is made up of a splint (1) fitting in the user's teeth, said splint incorporating a rear arch (2).

The angle α of said rear arch can be between 30 and 120 degrees with respect to the axis of the splint itself.

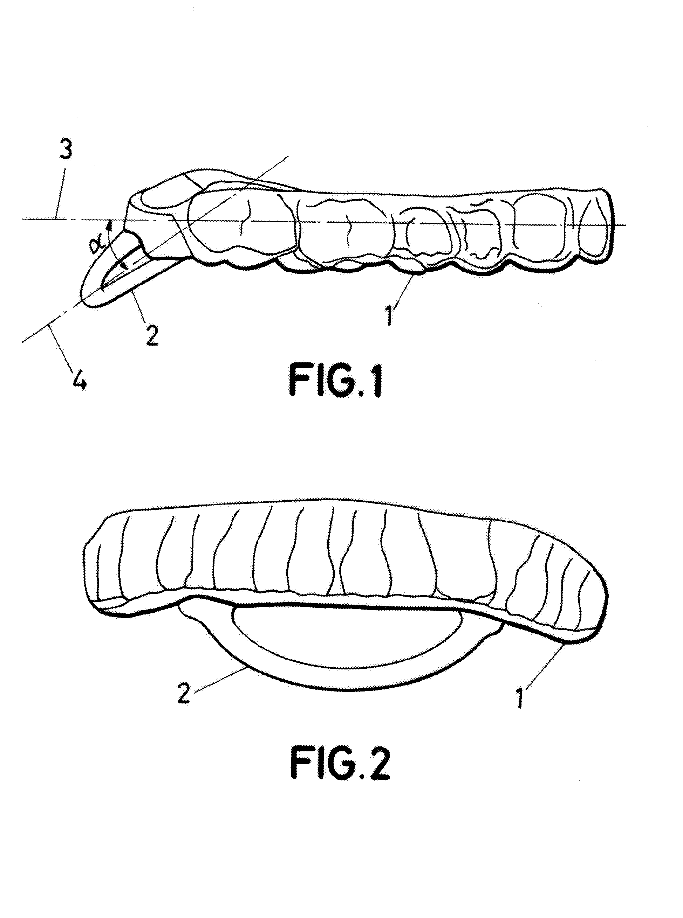

In Figure 1 showing a side view of the splint the axes of the splint (3) and of the arch (4), as well as the angle α formed between both axes are observed.

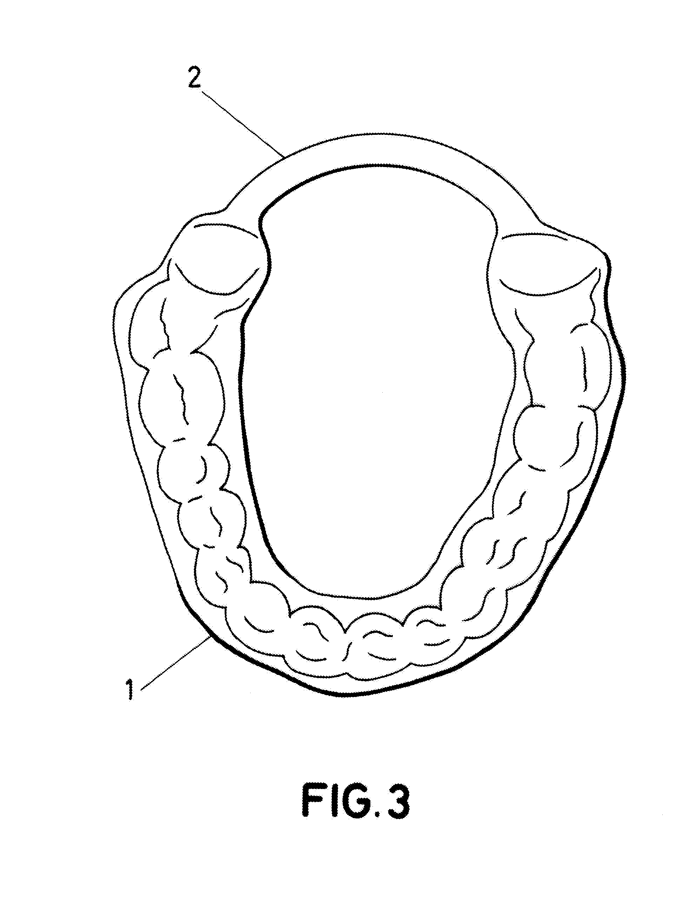

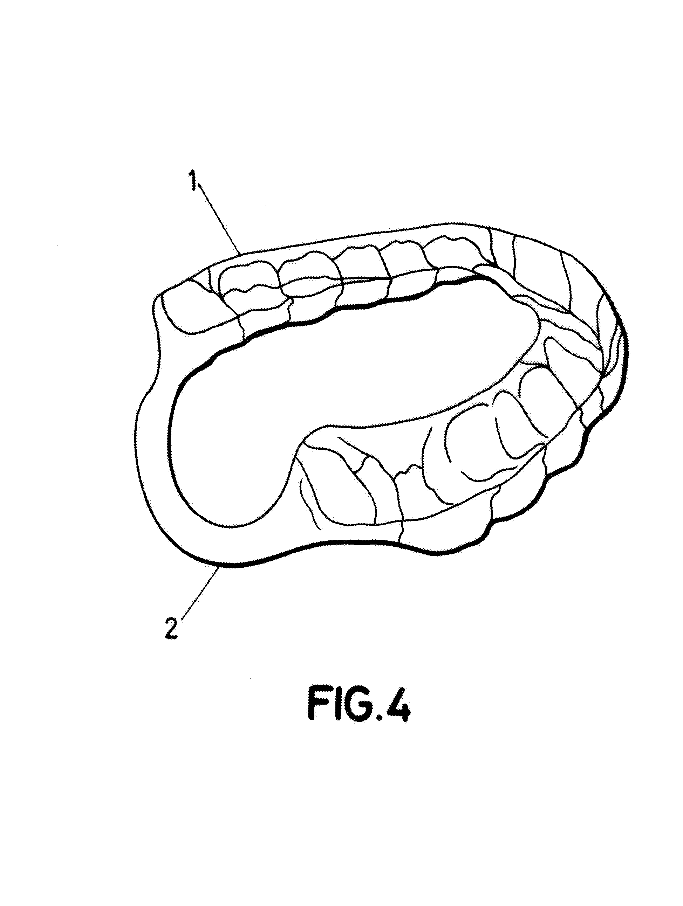

Figures 2, 3 and 4 show different views depicting the splint (1) and the rear arch (2) incorporated in the splint (1). As is observed in Figures 3 and 4, this splint could be made such that it is adapted to the shape of the user's teeth for greater comfort or if the person lacks teeth, it could be adapted to the mandible, the placement of oral adhesive preventing the movement of the splint being necessary in the latter case for better securing.

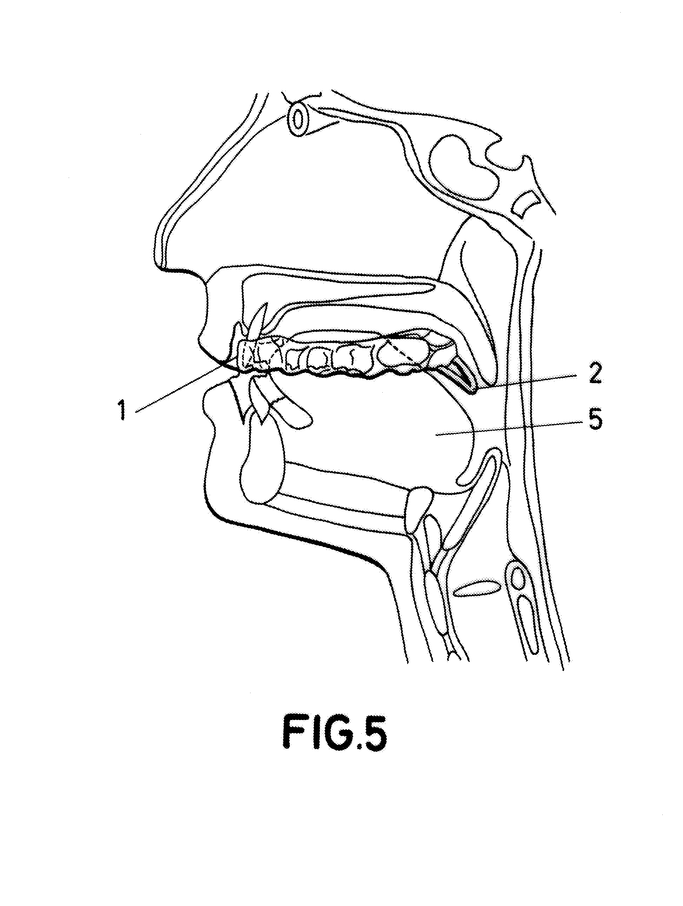

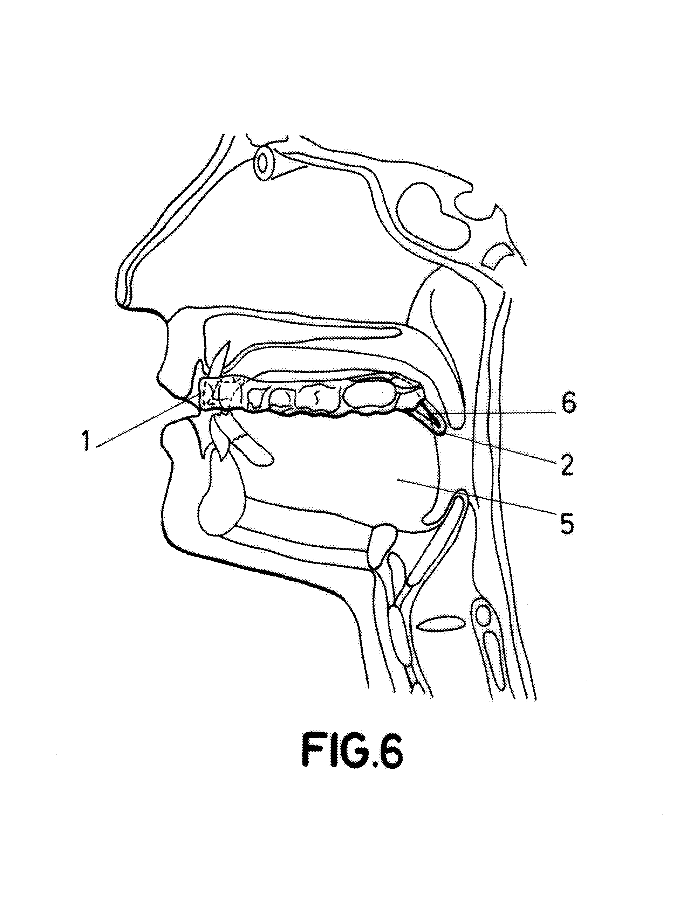

Figures 5 and 6 show a section of the oral cavity detailing the tongue (5) and, as shown in Figure 5, the tongue is in the rest position and does not press against the posterior pharyngeal wall and as shown in Figure 6, the tongue (5) is shifted towards its rear part making contact at the point (6) with the arch (2) of the splint (1) which prevents the tongue from shifting further towards its rear part, leaving the passage of the nasal airway free.

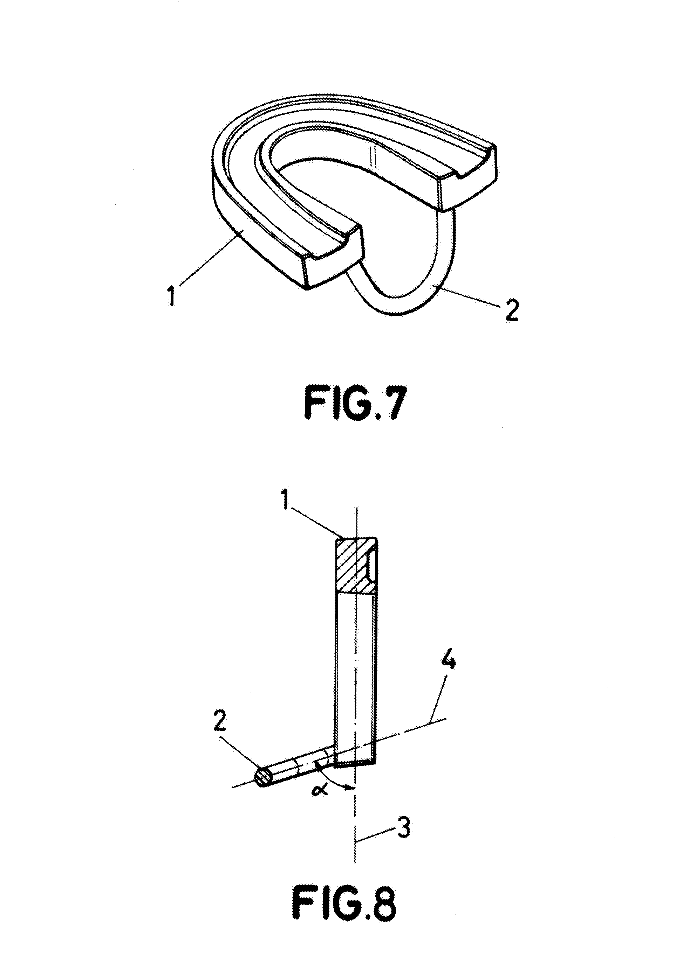

Figure 7 shows a perspective view of another splint (1) in a more simplified form and without the shape of the user's teeth in which an inclined arch (2) emerges from its rear part, such inclination being seen in greater detail in Figure 8 where the angle α formed between the axis of the splint (3) and the axis of the arch (4) is observed.

Though not shown in any of the drawings, the arch (2) can be divided into two halves in order to facilitate the process of manufacturing it, or the arch (2) can be continuous but the splint (1) can be divided in order to be able to be adapted to different mouth sizes.

## Claims

1. Intraoral splint (1) configured to be fitted to the upper user's teeth and comprising a rear arch (2) which extends from one rear end of the splint (1) to the opposed rear end of the splint (1); **characterised in that** said rear arch (2) has a negative slope angle (α) formed between the axis of the splint (3) and the axis of the arch (4), and **in that** said angle (α) ranges between 30 and 120 degrees with respect to the axis of the splint (3) itself.

2. Intraoral splint (1) according to claim 1, **characterized in that** the arch (2) is continuous and joins the two rear ends of the splint.

3. Intraoral splint (1) according to claim 1, **characterized in that** the arch (2) is discontinuous.

4. Intraoral splint (1) according to claim 1, **characterized in that** the arch (2) is continuous and the splint (1) is divided for adapting to different mouth sizes.

5. Intraoral splint according to claims 1 to 4, **characterized in that** the section of the arch (2) is circular or oval shaped without ridges or sharp edges damaging oral tissues.

6. Intraoral splint according to claims 1 to 4, **characterized in that** the section of the arch (2) has a flattened shape without ridges or sharp edges damaging oral tissues.

7. Intraoral splint according to claim 5, **characterized in that** the diameter of the section of the arch ranges between 1 and 20 mm.

8. Intraoral splint according to claims 1 to 6, **characterized in that** the size of the arch ranges between 10 and 60 mm.

## Patentansprüche

1. Intraorale Schiene (1), welche ausgebildet ist, um in die oberen Zähne eines Benutzers eingepasst zu werden, und welche einen hinteren Bogen (2) umfasst, welcher sich von einem hinteren Ende der Schiene (1) zum gegenüberliegenden hinteren Ende der Schiene (1) erstreckt; **dadurch gekennzeichnet, dass** der hintere Bogen (2) einen Winkel (α) mit negativer Steigung aufweist, welcher zwischen der Schienenachse (3) und der Bogenachse (4) gebildet ist, und dass der Winkel (α) zwischen 30 und 120 Grad bezüglich der Achse (3) der Schiene selbst beträgt.

2. Intraorale Schiene (1) nach Anspruch 1, **dadurch gekennzeichnet, dass** der Bogen (2) durchgehend ist und die beiden hinteren Enden der Schiene verbindet.

3. Intraorale Schiene (1) nach Anspruch 1, **dadurch gekennzeichnet, dass** der Bogen (2) unterbrochen ist.

4. Intraorale Schiene (1) nach Anspruch 1, **dadurch gekennzeichnet, dass** der Bogen (2) durchgehend ist und die Schiene (1) geteilt ist, um sich an unterschiedliche Mundgrößen anzupassen.

5. Intraorale Schiene nach den Ansprüchen 1 bis 4, **dadurch gekennzeichnet, dass** der Abschnitt des Bogens (2) kreisförmig oder oval geformt ist ohne Kanten oder scharfe Ränder, welche das Mundgewebe beschädigen.

6. Intraorale Schiene nach den Ansprüchen 1 bis 4, **dadurch gekennzeichnet, dass** der Abschnitt des Bogens (2) eine abgeflachte Form hat ohne Kanten oder scharfe Ränder, welche das Mundgewebe beschädigen.

7. Intraorale Schiene nach Anspruch 5, **dadurch gekennzeichnet, dass** der Durchmesser des Abschnitts des Bogens zwischen 1 und 20 mm beträgt.

8. Intraorale Schiene nach den Ansprüchen 1 bis 6, **dadurch gekennzeichnet, dass** die Größe des Bogens zwischen 10 und 60 mm beträgt.

## Revendications

1. Attelle intraorale (1) conçue pour être posée sur la mâchoire supérieure de l'utilisateur et comprenant un arc arrière (2) qui s'étend depuis une extrémité arrière de l'attelle (1) jusqu'à l'extrémité arrière opposée de l'attelle (1); **caractérisée en ce que** ledit arc arrière (2) a un angle d'inclinaison négatif (α) formé entre l'axe de l'attelle (3) et l'axe de l'arc (4), et **en ce que** ledit angle (α) est compris entre 30 et 120 degrés par rapport à l'axe de la propre attelle (3).

2. Attelle intraorale (1) selon la revendication 1, **caractérisée en ce que** l'arc (2) est continu et rejoint les deux extrémités arrière de l'attelle.

3. Attelle intraorale (1) selon la revendication 1, **caractérisée en ce que** l'arc (2) est discontinu.

4. Attelle intraorale (1) selon la revendication 1, **caractérisée en ce que** l'arc (2) est continu et l'attelle (1) est divisée pour s'adapter aux différentes tailles de bouche.

5. Attelle intraorale selon les revendications 1 à 4, **caractérisée en ce que** la section de l'arc (2) est de forme circulaire ou ovale sans stries ou bords coupants qui endommagent les tissus oraux.

6. Attelle intraorale selon les revendications 1 à 4, **caractérisée en ce que** la section de l'arc (2) est de forme aplatie sans stries ou bords coupants qui endommagent les tissus oraux.

7. Attelle intraorale selon la revendication 5, **caractérisée en ce que** le diamètre de la section de l'arc est compris entre 1 et 20 mm.

8. Attelle intraorale selon les revendications 1 à 6, **caractérisée en ce que** la taille de l'arc est comprise entre 10 et 60 mm.
